# EUROPÄISCHE PATENTSCHRIFT

(11) **EP 1 872 714 B1**
(45) Veröffentlichungstag und Bekanntmachung des Hinweises auf die Patenterteilung: **07.01.2009**
(21) Anmeldenummer: 06116245.9
(22) Anmeldetag: 28.06.2006
(51) Int. Cl.: A61B 5/00

(54) **Verfahren zur Überwachung einer Anordnung zur Bestimmung der Konzentration eines Analyten in einer Körperflüssigkeit**
Method for monitoring a system for the determination of analyte concentration in a body fluid
Procédé pour surveiller une système de la détermination de la concentration d'une analyte dans un fluid corporel

(43) Veröffentlichungstag der Anmeldung: 02.01.2008
(73) Patentinhaber: F. Hoffmann-La Roche AG, 4070 Basel (CH); Roche Diagnostics GmbH, 68305 Mannheim (DE)
(72) Erfinder: Rinne, Helmut, 22587 Hamburg (DE); Ocvirk, Gregor, 55283 Nierstein (DE)
(74) Vertreter: Huhn, Michael

(56) Entgegenhaltungen:
- EP-A2- 0 523 463
- DE-A1- 4 405 149

## Beschreibung

Die Erfindung bezieht sich auf ein Überwachungsverfahren für eine Anordnung zur Bestimmung der Konzentration eines Analyten in einer Körperflüssigkeit in dem Körper eines lebenden Menschen oder Tieres und auf eine solche Anordnung mit entsprechenden Überwachungsmitteln.

Anordnungen dieser Art werden im Stand der Technik zum Beispiel zum Überwachen der Glucosekonzentration im Blut eines Menschen verwendet. EP 0 722 288 B1 hat ein Verfahren und eine Vorrichtung zum Überwachen der Konzentration einer ausgewählten Substanz oder einer ausgewählten Gruppe von Substanzen in einer Körperflüssigkeit im Körper eines lebenden Menschen oder Tieres zum Gegenstand. Dabei wird die zu überwachende Substanz oder die Gruppe von Substanzen vom Körper aus durch eine Schnittstelle geleitet und von der Rückseite der Schnittstelle in einer Perfusionsflüssigkeitsströmung wegtransportiert. Die Konzentration der zu überwachenden Substanz oder der Gruppe von Substanzen wird in der Perfusionsflüssigkeitsströmung stromabwärts der Schnittstelle gemessen, wobei die Strömungsrate der Perfusionsflüssigkeitsströmung weniger als 60 µl/h beträgt.

DE 44 05 149 C2 betrifft eine Anordnung zur Bestimmung der Konzentration von Inhaltsstoffen in Körperflüssigkeiten mit einer im Körpergewebe implantierbaren und mit einer Perfusatlösung durchströmten Dialysesonde, die zuflussseitig über eine Pumpe mit einem Perfusatlösungsreservoir und rückflussseitig über mindestens eine als Sensor für die Inhaltsstoffe-Konzentration dienende Enzymzelle mit einem Auffangbehälter verbunden ist. Die Enzymzelle ist ausgangsseitig mit einer extrakorporal angebrachten Auswerte-/Anzeigeeinheit verbunden. Das Perfusatlösungsreservoir, die Pumpe, die Enzymzelle und der Auffangbehälter sind dabei getrennt von der Auswerte-/Anzeigeeinheit als Sensoreinheit in einem eigenen ersten Gehäuse zusammengefasst. Die Dialysesonde, das Perfusatlösungsreservoir, die Pumpe, die Enzymzelle und der Auffangbehälter sind getrennt von der Auswerte-/Anzeigeeinheit als Sonden-/Sensoreinheit in einem eigenen zweiten Gehäuse zusammengefasst.

In solchen im Stand der Technik bekannten Anordnungen kann eine Vielzahl von Funktionsstörungen auftreten, die negative Auswirkungen auf die Messgenauigkeit haben. Ein fehlerhaft bestimmter Konzentrationswert des Analyten kann jedoch gefährliche Folgen für den Benutzer der Anordnung haben, der zum Beispiel die Verabreichung von Insulin in Abhängigkeit von dem bestimmten Glucose-Konzentrationswert steuert. Folglich ist eine zuverlässige Überwachung der Anordnung zur Bestimmung der Konzentration des Analyten erforderlich.

Die Aufgabe der Erfindung besteht daher darin, die Nachteile des Standes der Technik zu vermeiden, und insbesondere ein Verfahren zur zuverlässigen Überwachung einer Anordnung zur Bestimmung der Konzentration eines Analyten in einer Körperflüssigkeit zur Verfügung zu stellen.

Diese Aufgabe wird erfindungsgemäß gelöst durch ein Verfahren zur Überwachung einer Anordnung zum Bestimmen einer Konzentration eines Analyten in einer Körperflüssigkeit, wobei das Bestimmen der Konzentration des Analyten mittels der Anordnung darauf beruht, dass der Analyt aus der Körperflüssigkeit durch eine Schnittstelle hindurch tritt und in einer Flüssigkeitsströmung in eine Durchflussmesskammer transportiert wird, in der eine Messung zur Bestimmung der Konzentration des Analyten durchgeführt wird, und eine Auswertung der Messung in einer Signalverarbeitungseinrichtung erfolgt. Die Überwachung der Anordnung umfasst die Schritte
- Messen von Messwerten mindestens zweier miteinander korrelierter Systemparameter der Anordnung mittels eines Sensorsystems und
- Vergleichen der Messwerte mit jeweils für die Systemparameter in einer Speichereinheit hinterlegten Grenzwerten zum Erhalt einer Kombination von mindestens zwei Vergleichsergebnissen.

Die zu überwachende Anordnung umfasst eine Schnittstelle, über die der Analyt (zum Beispiel Glucose) aus der Körperflüssigkeit (zum Beispiel Blut oder interstitieller Flüssigkeit) in die Anordnung gelangen kann. Die Körperflüssigkeit, in der die Konzentration des Analyten durch die Anordnung bestimmt wird, kann direkt mit der Schnittstelle in Kontakt stehen, oder die Konzentration in der Körperflüssigkeit kann indirekt aus der Konzentration des Analyten in der mit der Schnittstelle in Kontakt stehenden Flüssigkeit berechnet werden. Die Schnittstelle ist beispielsweise eine semipermeable Membran in einer Mikrodialysesonde, die subkutan in das Gewebe eines Körpers eingebracht wird. Die semipermeable Membran trennt den interstitiellen Raum von dem Innenraum der Sonde und erlaubt die Diffusion des Analyten aus der interstitiellen Flüssigkeit in eine Perfusionsflüssigkeit im Inneren der Sonde. Als Schnittstelle der zu überwachenden Anordnung kann jedoch eine beliebige Schnittstelle dienen, die ein Eintreten des Analyten in die Anordnung erlaubt, insbesondere auch ein offenes Fenster.

Der durch die Schnittstelle hindurchgetretene Analyt wird in einer Flüssigkeitsströmung (Perfusionsflüssigkeitsströmung / Dialysatströmung) in eine Durchflussmesskammer transportiert. Die Perfusionsflüssigkeit (zum Beispiel eine isotone analytfreie Flüssigkeit) wird vorzugsweise durch eine Transportvorrichtung (insbesondere eine Pumpe, zum Beispiel eine mikromechanische Membranpumpe oder eine Rollenpumpe) aus einem Reservoir zu der Schnittstelle und von der Schnittstelle aus mit dem darin enthaltenen Analyten durch eine Flüssigkeitsleitung zu einer Durchflussmesskammer transportiert.

In der Durchflussmesskammer erfolgt eine Messung zur Bestimmung der Konzentration des Analyten. Dazu ist in der Messkammer eine Detektoranordnung enthalten. Vorteilhafterweise ist die Detektoranordnung als amperometrisch arbeitende Detektoranordnung ausgebildet. Amperometrie ist eine elektrochemische Methode, die auf einer Strommessung bei konstantem Potenzial basiert. Dazu werden vorzugsweise die im Stand der Technik bekannte "3-Elektroden-Methode" und ein Potentiostat eingesetzt.

Zur Bestimmung der Glucosekonzentration in einer Flüssigkeit wird beispielsweise mit Hilfe des Enzyms Glucoseoxidase die Glucose unter anderem in Wasserstoffperoxid umgesetzt. Das gebildete Wasserstoffperoxid dient als Nachweismolekül und wird an einer von der Detektoranordnung umfassten Arbeitselektrode oxidiert und der dabei erzeugte elektrische Strom detektiert. Dazu wird eine Dreielektrodenmessanordnung , bestehend aus einer zu regelnden Arbeitselektrode, einer Gegenelektrode und einer Referenzelektrode, d.h. einer Elektrode konstanten elektrochemischen Potenzials eingesetzt. Das für die gewünschte Reaktion erforderliche Potenzial zwischen Arbeits- und Referenzelektrode wird durch Regelung des Stromes zwischen Arbeits- und Gegenelektrode durch den Potentiostaten eingestellt.

Die Auswertung der mit der Detektoranordnung in der Durchflussmesskammer durchgeführten Messung (zum Beispiel des gemessenen Stroms, der bei der Oxidation des Wasserstoffperoxids an der Arbeitselektrode erzeugt wird) erfolgt mittels einer in der Anordnung enthaltenen Signalverarbeitungseinrichtung. Die Signalverarbeitungseinrichtung bestimmt aus den Messwerten der Detektoranordnung die Konzentration des Analyten in der Körperflüssigkeit. Die verbrauchte Flüssigkeit wird von der Durchflussmesskammer vorzugsweise in einen in der Anordnung enthaltenen Auffangbehälter transportiert und in diesem gespeichert.

Eine Fehlfunktion einer solchen Anordnung zum Bestimmen der Konzentration eines Analyten in einer Körperflüssigkeit kann sich auf eine Vielzahl von Systemparametern auswirken. Der Begriff "Systemparameter" bezieht sich im Zusammenhang mit der Erfindung auf Parameter, die von dem aktuellen Ist-Zustand der Anordnung abhängen und die Funktionsfähigkeit, insbesondere die Genauigkeit der mit der Anordnung bestimmten Messwerte, beeinflussen. Beispiele für Systemparameter sind die Temperatur der Flüssigkeitsströmung, die durch die Durchflussmesskammer strömt, die elektrische Spannung zwischen Arbeits- und Gegenelektrode und zwischen Arbeits- und Referenzelektrode einer in der Durchflussmesskammer vorhandenen Detektoranordnung, die Durchflussrate der Perfusionsflüssigkeit durch die Durchflussmesskammer, Pumpenparameter einer zum Pumpen der Perfusionsflüssigkeit entlang der Schnittstelle und durch die Durchflussmesskammer vorgesehenen Pumpe, wie der elektrische Pumpenstrom und die Drehzahl der Pumpe, die Batteriespannung einer zur Energieversorgung der Anordnung vorgesehenen Batterie und die aktive Membranaustauschfläche einer als Schnittstelle vorgesehenen Membran.

Um eine Fehlfunktion der Anordnung rechtzeitig zu erkennen und sich daraus ergebende Gefahren zu vermeiden (zum Beispiel die Verabreichung einer zu hohen oder zu niedrigen Insulindosis an einen Diabetiker) wird die Anordnung mittels des erfindungsgemäßen Verfahrens überwacht.
Zur Überwachung der Anordnung ist ein Sensorsystem in der Anordnung vorgesehen. Mittels dieses Sensorsystems werden erfindungsgemäß Messwerte mindestens zweier miteinander korrelierter Systemparameter der Anordnung gemessen. Miteinander korrelierte Systemparameter sind in diesem Zusammenhang Systemparameter, deren Werte zusammenhängen.

Gemäß der Erfindung werden die Messwerte der mindestens zwei miteinander korrelierten Systemparameter mit jeweils für die mindestens zwei Parameter in einer Speichereinheit der Anordnung hinterlegten Grenzwerten verglichen (vorzugsweise erfolgt der Vergleich von zwei oder drei miteinander korrelierten Systemparametern). Die Grenzwerte können für jeden Systemparameter eine Obergrenze und/oder eine Untergrenze definieren. Als Vergleichsergebnisse sind dann für jeden Systemparameter denkbar:
- Der Messwert des Systemsparameters liegt über der Obergrenze oder
- der Messwert liegt unter der Obergrenze oder
- der Messwert liegt unter der Obergrenze und über der Untergrenze oder
- der Messwert liegt über der Untergrenze oder
- der Messwert liegt unter der Untergrenze.

Aus dem Vergleichen der Messwerte der mindestens zwei miteinander korrelierten Systemparameter mit den Grenzwerten ergibt sich eine bestimmte Kombination von Vergleichsergebnissen. Beispielsweise könnte sich aus den Messwerten zweier miteinander korrelierter Systemparameter die folgende Kombination aus zwei Vergleichsergebnissen ergeben:
1. Der Messwert des ersten Systemparameters liegt über einer definierten Obergrenze.
2. Der Messwert des zweiten Systemparameters liegt zwischen einer definierten Unter- und einer definierten Obergrenze.

Dieser Kombination von mindestens zwei Vergleichsergebnissen kann ein bestimmter Zustand der Anordnung zugeordnet werden. Zustände, die bestimmten Kombinationen von Vergleichsergebnissen zugeordnet werden können, sind Zustände der Anordnung, die ihre Funktionsfähigkeit betreffen. Dabei können solche Zustände zugeordnet werden, die sich in der Kombination der gemessenen Systemparameter niederschlagen. Mögliche Zustände, die der Anordnung zugeordnet werden können, sind vorzugsweise ausgewählt aus der Gruppe, dass die korrekte Funktion der Anordnung gewährleistet ist, dass die Effizienz einer Komponente der Anordnung herabgesetzt ist, dass ein bestimmter Defekt der Anordnung erkannt wird, oder dass ein unbekannter Defekt der Anordnung vorliegt.

Die Aussage der Messungen der mindestens zwei miteinander korrelierten Systemparameter zusammen ermöglicht ein Erkennen des Zustandes der Anordnung, insbesondere bestimmter Defekte der Anordnung, welches die Messung eines Systemsparameters allein nicht zulässt. Somit ist eine zuverlässige kontinuierliche Überwachung der Funktionsfähigkeit der Anordnung durch das erfindungsgemäße Verfahren möglich.

Die Erfindung bezieht sich weiterhin auf eine Anordnung zum Bestimmen einer Konzentration eines Analyten in einer Körperflüssigkeit, umfassend eine Schnittstelle zum Aufnehmen des Analyten aus einer Körperflüssigkeit in eine Perfusionsflüssigkeitsströmung in der Anordnung, eine Flüssigkeitsleitung zum Leiten der Flüssigkeitsströmung von der Schnittstelle zu einer Durchflussmesskammer, eine Transportvorrichtung zum Transport der Flüssigkeitsströmung durch die Flüssigkeitsleitung von der Schnittstelle zu der Durchflussmesskammer, eine Detektoranordnung in der Durchflussmesskammer zur Durchführung von Messungen zur Bestimmung der Konzentration des Analyten und eine Signalverarbeitungseinrichtung zur Berechnung der Konzentration des Analyten aus Ergebnissen der Messung durch die Detektoranordnung. Die Signalverarbeitungseinrichtung ist vorzugsweise so ausgebildet, dass sie einen Referenzwert aufnehmen und zur Kalibration verrechnen kann. Die erfindungsgemäße Anordnung enthält weiterhin ein Sensorsystem zum Messen von Messwerten mindestens zweier (insbesondere mindestens dreier) miteinander korrelierter Systemparameter, eine Speichereinheit und Mittel (insbesondere einen Prozessor) zum Vergleichen der Messwerte mit jeweils für die Systemparameter in der Speichereinheit hinterlegten Grenzwerten zum Erhalt einer Kombination von mindestens zwei (insbesondere mindestens drei) Vergleichsergebnissen.

Diese erfindungsgemäße Anordnung dient zur Durchführung des erfindungsgemäßen Verfahrens. Die erfindungsgemäße Anordnung ist beim Gebrauch vorzugsweise mit Ausnahme der Schnittstelle (zum Beispiel Mikrodialysesonde mit semipermeabler Membran) und gegebenenfalls einem kleinen Teil der sich zu der Schnittstelle und von der Schnittstelle erstreckenden Flüssigkeitsleitungen außerhalb des Körpers eines lebenden Menschen oder Tieres angeordnet. Insbesondere wird das erfindungsgemäße Überwachungsverfahren ausschließlich außerhalb des Körpers durchgeführt. Die Sensoren des Sensorsystems sind dazu stromaufwärts von, stromabwärts von und/oder in der Durchflussmesskammer angeordnet. Die gesamte erfindungsgemäße Anordnung ist vorzugsweise weitgehend miniaturisiert und es handelt sich um eine tragbare Anordnung. Vorzugsweise umfasst das Sensorsystem mindestens zwei Sensoren, ausgewählt aus der Gruppe elektrischer Spannungssensor, elektrischer Stromsensor, Temperatursensor, Flussratensensor, Drehzahlmesser und Konzentrationssensor. Der Konzentrationssensor ist insbesondere zur Messung der Konzentrationsänderung eines Stoffaustauschmarkers vorgesehen.

Die Konzentrationsbestimmung des Analyten erfolgt bei der erfindungsgemäßen Anordnung vorzugsweise auf elektrochemisch-amperometrischem Wege, wobei der Messsensor eine in einer Durchflussmesskammer angeordnete polarisierbare Elektrodenanordnung umfasst. Diese kann zum Beispiel aus einer Arbeitselektrode aus Platin, einer Gegenelektrode aus Silber oder Platin und einer Referenzelektrode aus Silber oder Silber/Silberchlorid bestehen.

Gemäß einer bevorzugten Ausführungsform der vorliegenden Erfindung löst die Signalverarbeitungseinrichtung bei definierten Kombinationen von Vergleichsergebnissen mindestens eine Reaktion der Anordnung aus, wobei die mindestens eine Reaktion ausgewählt ist aus der Gruppe
- Anzeigen einer Warnung oder Problemmeldung durch eine Anzeigeeinrichtung der Anordnung,
- Anzeigen einer Rekalibrationsaufforderung durch die Anzeigeeinrichtung,
- Anzeigen einer Aufforderung, eine Komponente der Anordnung auszutauschen, durch die Anzeigeeinrichtung,
- Abschalten der Anordnung,
- Abgabe eines optischen, akustischen oder haptischen Alarmsignals,
- Regeln mindestens eines Systemsparameters,
- Beeinflussen von Werten eines ersten Systemparameters durch Regeln von Werten eines zweiten, mit dem ersten Systemparameter korrelierten Systemparameters,
- kurzzeitiges Ändern eines Systemparameters zur Behebung eines aufgrund der Kombination der Vergleichsergebnisse möglichen Systemsfehlers.

Die Anordnung umfasst vorzugsweise eine Anzeigeeinrichtung (zum Beispiel ein Flüssigkristalldisplay), die neben Warnungen zum Beispiel auch mit der Anordnung bestimmte Konzentrationswerte oder Benutzermenues anzeigen kann, aus denen ein Benutzer Benutzermenuepunkte zum Beispiel mittels einer Tastatur oder einer sonstigen Bedieneinheit auswählen kann. Bei bestimmten Kombinationen von Vergleichsergebnissen kann die Anzeigeeinrichtung Warnungen anzeigen, die einen Benutzer zum Beispiel vor einem Defekt der Anordnung oder einer daraus gegebenenfalls folgenden fehlerhaften Konzentrationswertbestimmung warnen. Ergibt sich zum Beispiel die folgende Kombination von Vergleichsergebnissen:
a) Der Messwert der Gegenspannung (erster Systemparameter) zwischen Gegen- und Arbeitselektrode befindet sich außerhalb eines für diesen Systemparameter durch eine Obergrenze und eine Untergrenze definierten Bereichs,
b) die Temperatur der Flüssigkeitsströmung (zweiter, mit dem ersten Systemparameter korrelierter Systemparameter) liegt unter einer definierten Obergrenze und über einer definierten Untergrenze und
c) die Durchflussrate durch die Durchflussmesskammer (dritter, mit dem ersten Systemparameter korrelierter Systemparameter) liegt unter einer definierten Obergrenze und über einer definierten Untergrenze,
so zeigt die Anzeigeeinrichtung vorzugsweise eine Warnung vor einem Systemfehler an.

Ferner kann die erfindungsgemäße Anordnung bei definierten Kombinationen von Vergleichsergebnissen (insbesondere bei solchen Kombinationen, die auf einen Defekt der Anordnung schließen lassen) ein optisches Alarmsignal (zum Beispiel Blinken einer LED), ein akustisches Alarmsignal (zum Beispiel Abgabe eines Warntons) und/oder ein haptisches Alarmsignal (zum Beispiel Vibrieren einer Komponente der Anordnung) abgeben.

Eine weitere mögliche Reaktion der Anordnung auf bestimmte Kombinationen von Vergleichsergebnissen ist das Anzeigen einer Aufforderung zur Rekalibration durch die Anzeigeeinrichtung. Eine Rekalibration kann durch eine direkte Messung der Konzentration des Analyten in einer entnommenen Probe der Körperflüssigkeit und ein Eingeben dieses direkt gemessenen Konzentrationswertes in die Anordnung als Kalibrationswert erfolgen. Eine solche Kalibration ist zum Beispiel erforderlich, wenn ein aktuell gestörter Zustand der Anordnung, welcher zu einem unzulässig hohem Messfehler führt, nicht durch eine Regelung eines Systemparameters in einen ungestörten Zustand, welcher einen zulässig hohen Messfehler zur Folge hat, überführt werden kann. Eine weitere mögliche Reaktion der Anordnung auf bestimmte Kombinationen von Vergleichsergebnissen ist das Anzeigen einer Aufforderung, eine Komponente der Anordnung auszutauschen, durch die Anzeigeeinrichtung. Beispielsweise kann der Benutzer zum Austauschen einer Batterie aufgefordert werden.

Weiterhin können bei der vorliegenden Erfindung als Reaktion auf eine definierte Kombination von Vergleichsergebnissen ein Regeln eines Systemparameters und/oder ein Beeinflussen von Werten eines ersten Systemparameters durch das Regeln von Werten eines zweiten, mit dem ersten Systemparameter korrelierten Systemparameters vorgesehen sein. Dazu sind in der erfindungsgemäßen Anordnung Regeleinrichtungen vorgesehen. Beispielsweise kann die sich unter dem Grenzwert befindliche Flussrate mithilfe der Pumpdrehzahl geregelt werden. Im Falle einer nicht erfolgreichen Regelung ergibt sich hieraus eine veränderte Sensitivität der Strom/Konzentrationskurve, sodass z.B. eine Rekalibrationsaufforderung erfolgt.

Ferner kann die Signalverarbeitungseinrichtung in bestimmten Fällen ein Abschalten der Anordnung veranlassen. Beispielsweise kann die Signalverarbeitungseinrichtung das Abschalten der Anordnung veranlassen, wenn nach temporärer Erhöhung der Förderrate, die über eine Stunde gemessene mittlere Flussrate den gespeicherten Flussratengrenzwert unterschreitet.

Eine weitere mögliche Reaktion der Anordnung auf bestimmte Kombinationen von Vergleichsergebnissen ist eine kurzzeitige Änderung eines Systemparameters zur Behebung eines aufgrund der Kombination der Vergleichsergebnisse möglichen Systemfehlers. Beispielsweise kann bei der folgenden Kombination von Vergleichsergebnissen
a) der Messwert der Durchflussrate liegt unter einer definierten Untergrenze,
b) der Messwert der Batteriespannung liegt über einer definierten Untergrenze und
c) der Messwert einer Pumpenvariable (zum Beispiel der Drehzahl der Pumpe) befindet sich unter einer definierten Obergrenze,
kurzzeitig (für eine definierte Zeitspanne Δt) die Pumpenvariable (zum Beispiel die Drehzahl der Pumpe) erhöht werden, um ein möglicherweise vorhandenes Flusshindernis in den Leitungen für die Perfusionsflüssigkeit wegzuspülen. Nach Verstreichen der Zeitspanne wird die Pumpenvariable wieder gesenkt und erneut ein Durchflussratenmesswert aufgenommen, der mit den für die Durchflussrate definierten Grenzwerten verglichen wird.

Bei dem erfindungsgemäßen Verfahren führt das Sensorsystem vorzugsweise Messungen zur Bestimmungen mindestens zweier Systemparameter, ausgewählt aus der Gruppe
- Temperatur der Flüssigkeitsströmung,
- elektrische Spannung zwischen mindestens zwei Elektroden, an denen die Flüssigkeitsströmung in der Durchflussmesskammer entlangströmt,
- zeitliche Änderung eines elektrischen Stroms im Verhältnis zu der zeitlichen Änderung einer elektrischen Spannung in Bezug auf mindestens zwei in der Durchflussmesskammer angeordnete Elektroden,
- Systemvariable einer in der Anordnung enthaltenen Transportvorrichtung, die zum Transport der Flüssigkeitströmung dient,
- Batteriespannung mindestens einer in der Anordnung zur Energieversorgung vorgesehenen Batterie,
- Durchflussrate der Flüssigkeitsströmung durch die Durchflussmesskammer und
- Konzentration eines Markers
durch.

Die Temperatur der Flüssigkeitsströmung kann zum Beispiel direkt mittels eines Temperatursensors gemessen werden, der in der Flüssigkeitsströmung direkt vor, in oder direkt hinter der Durchflussmesskammer positioniert ist. Sie kann aber auch indirekt durch eine Temperaturmessung mittels des Temperatursensors erfasst werden, wobei der Temperatursensor die Temperatur eines mit der Flüssigkeitsströmung direkt oder indirekt in Kontakt stehenden Bauteils misst. Die in einer elektrochemischen Durchflussmesskammer zur Konzentrationsbestimmung von Analyten ablaufende elektrochemische Umsetzung ist unter anderem aufgrund der Temperaturabhängigkeit des Stofftransports von Analyten, Reaktanden und Reaktionsprodukten von und zur Elektrode sowie der Geschwindigkeitskonstanten der Reaktion selbst, von der Temperatur der Perfusionsflüssigkeit (des Dialysates) in der Durchflussmesskammer abhängig. Daher ist es zum Beispiel einerseits bei bekannter Temperaturabhängigkeit des Messsignals möglich, die Temperatur zu messen und auftretende Temperaturschwankungen rechnerisch zu kompensieren, oder andererseits die Temperaturschwankungen durch Thermostatisierung zu minimieren.

Die elektrische Spannung zwischen einer Gegen- und einer Arbeitselektrode (Gegenspannung) und/oder zwischen einer Referenz- und einer Arbeitselektrode (Zellspannung) kann bestimmt werden, wobei die Gegen-, Referenz- und Arbeitselektroden zur Konzentrationsmessung eines Analyten (zum Beispiel von Glucose) vorgesehen sind. Die für die elektrochemische Umsetzung des Analyten angelegte Zellspannung ist so zu wählen, dass der gemessene Strom der elektrochemischen Umsetzung möglichst weitgehend auf die Umsetzung des Analyten an der Arbeitselektrode zurückzuführen ist, d. h. dass die Umsetzung von Interferenzen möglichst gering gehalten werden kann. Hierzu ist es erforderlich, dass die Zellspannung während des Betriebes der Anordnung möglichst konstant gehalten wird.

Eine in der Anordnung enthaltene Transportvorrichtung, die zum Transport der Perfusionsflüssigkeit dient, ist beispielsweise eine Pumpe, die als Membranpumpe (z.B. piezoelektrisch angetrieben), Kolbenpumpe (z.B. Spritzenpumpe), dynamische Pumpe (z.B. hydrodynamische oder elektroosmotische Pumpe) oder auch als Schlauchpumpe ausgeführt werden kann. Systemvariablen einer Pumpe sind zum Beispiel der elektrische Pumpenstrom oder die Drehzahl der Pumpe.

Die mindestens eine in der Anordnung vorgesehene Batterie (oder ein Akku) dient zum Beispiel der Energieversorgung einer als Transportvorrichtung für die Perfusionsflüssigkeit vorgesehenen, elektrisch betriebenen Pumpe, einer Anzeigeeinrichtung, einer Signalverarbeitungseinrichtung und anderer Komponenten der erfindungsgemäßen Anordnung.

Zur Erfassung der Durchflussrate der Flüssigkeitsströmung durch die Durchflussmesskammer kann ein Flussratensensor vorgesehen sein, der zum Beispiel flussabwärts von der Durchflussmesskammer positioniert ist. Der transmembrane diffusive Stofftransport in einem (Mikrodialyse-)Katheter unterliegt einer nicht linearen Abhängigkeit von der Durchflussrate. Ferner ist in einer amperometrischen Durchflussmesskammer bei konstanter Analytkonzentration ein flussratenabhängiges Sensorsignal festzustellen. Schließlich hat die Durchflussrate unmittelbare Auswirkung auf den Zeitversatz zwischen der Anreicherung des Analyten im Perfusat und der Vermessung der Analytkonzentration in der ex-vivo-Durchflussmesskammer (Totzeit). Aufgrund dieser Zusammenhänge ist es erstrebenswert, die Systemdurchflussrate möglichst konstant zu halten. Innerhalb der technisch umsetzbaren Toleranzen der Systemdurchflussrate ist die Systemdurchflussrate ferner so zu wählen, dass bei geringfügiger Veränderung der Systemdurchflussrate nur geringfügige Änderungen des Messsignals resultieren.

Weiterhin kann die Konzentration eines Markers in der Flüssigkeitsströmung bestimmt werden, um eine Aussage über den transmembranen, diffusiven Stoffaustausch an der Schnittstelle der Anordnung (bei Vorliegen einer Membran) zu treffen. Der transmembrane, diffusive Flux ist bei gegebener Perfusionsflüssigkeit und Temperatur von der Durchflussrate durch eine Leitung (Katheter) entlang der Membran und von der Membranaustauschfläche abhängig. Die Variation der Membranaustauschfläche in vivo kann durch Konzentrationsbestimmung eines endogenen Markers, zum Beispiel von Natriumionen, der in nahezu konstanter Konzentration vorliegt, bestimmt werden. Der transmembrane, diffusive Flux des Analyten kann hieraus rechnerisch ermittelt werden, so dass zum Beispiel eine Anpassung der Durchflussrate zwecks Erhöhung des diffusiven Fluxes vorgenommen werden kann.

Je zwei miteinander korrelierte Systemparameter im Sinne der vorliegenden Erfindung sind insbesondere
a) die Durchflussrate und die Batteriespannung (da bei gesenkter Batteriespannung die Förderleistung der Pumpe und damit die Durchflussrate sinkt),
b) die Durchflussrate und eine Systemvariable der Transportvorrichtung (da eine Systemvariable der Transportvorrichtung direkt die Durchflussrate beeinflusst),
c) die Batteriespannung und eine Systemvariable der Transportvorrichtung (siehe Kommentare zu a und b),
d) die Gegenspannung und die Temperatur der Perfusionsflüssigkeit (bei erhöhter Temperatur erhöht sich der Stoffumsatz und somit die zum Erhalt einer konstanten Zellspannung erforderliche Gegenspannung),
e) die Gegenspannung und die Durchflussrate (bei erhöhter Flussrate sinkt der elektrochemische Stoffumsatz/Zeiteinheit in der Durchflussmesskammer und somit die Gegenspannung) und
f) das Verhältnis der zeitlichen Änderung des elektrischen Messstroms in der Durchflussmesskammer zu der zeitlichen Änderung der Gegenspannung und die Durchflussrate (wenn das Verhältnis unter einem bestimmten Grenzwert liegt, besteht der Verdacht einer Belegung der Arbeitselektrode durch Partikel oder eine Luftblase, die sich wiederum negativ auf die Durchflussrate auswirkt).

Gemäß einer bevorzugten Ausführungsform der vorliegenden Erfindung werden bei der Durchführung des erfindungsgemäßen Überwachungsverfahrens eine Gegenspannung zwischen einer in der Durchflussmesskammer angeordneten Gegen- und einer Arbeitselektrode, eine Temperatur der Flüssigkeitsströmung und eine Durchflussrate der Flüssigkeitsströmung durch die Durchflussmesskammer mittels der Signalverarbeitungseinrichtung mit in der Speichereinheit hinterlegten Grenzwerten verglichen.

Gemäß einer bevorzugten Ausführungsform der vorliegenden Erfindung werden bei der Durchführung des erfindungsgemäßen Überwachungsverfahrens eine Durchflussrate der Flüssigkeitsströmung durch die Durchflussmesskammer, eine Batteriespannung einer in der Anordnung enthaltenen Batterie und eine Systemvariable einer in der Anordnung enthaltenen Transportvorrichtung, die zum Transport der (Perfusions)flüssigkeitsströmung dient, durch die Signalverarbeitungseinrichtung mit in der Speichereinheit hinterlegten Grenzwerten verglichen.

Vorzugsweise werden Werte mindestens einer durch das Sensorsystem gemessenen Systemvariablen bei der Bestimmung der Konzentration des Analyten berücksichtigt. Beispielsweise kann die Temperatur der Flüssigkeitsströmung mittels eines Temperatursensors gemessen und dann aus dem Temperaturmesswert ein Kompensationswert ermittelt werden. Die Temperaturabhängigkeit des Elektrodensignals zur Konzentrationsbestimmung des Analyten kann sodann mittels des Konzentrationswertes rechnerisch eliminiert werden. Verschiedene Möglichkeiten zur Ermittlung des Kompensationswertes sind im Stand der Technik bekannt.

Gemäß einer bevorzugten Ausführungsform der vorliegenden Erfindung werden mit dem Sensorsystem zumindest zwei miteinander korrelierte Systemparameter kontinuierlich überwacht, während die Konzentrationsbestimmung des Analyten ebenfalls kontinuierlich erfolgt. Besonders bevorzugt erfolgt die Messung zur Bestimmung der Konzentration des Analyten mit einer definierten Messfrequenz und das Erfassen der Messwerte der mindestens zwei miteinander korrelierten Systemparameter ebenfalls mit dieser definierten Messfrequenz. Somit ist eine unmittelbare Zuordnung von Messwerten, die zur Bestimmung der Konzentration des Analyten herangezogen werden zu Werten der Systemparameter möglich.

Das erfindungsgemäße Verfahren kommt gemäß einer bevorzugten Ausführungsform zur Überwachung einer Anordnung zum Einsatz, die zum Bestimmen der Konzentration von Glucose in eine Körperflüssigkeit, insbesondere im Blut, eines lebenden Menschen eingesetzt wird. Die erfindungsgemäße Anordnung wird gemäß einer bevorzugten Ausführungsform zum Bestimmen der Konzentration von Glucose in einer Körperflüssigkeit eines lebenden Menschen verwendet. Anhand der Zeichnung wird die Erfindung nachstehend näher erläutert.

Es zeigt:
- Figur 1: eine schematische Darstellung einer erfindungsgemäßen Anordnung,
- Figur 2: ein Ablaufdiagramm zu einer ersten Ausführungsform eines erfindungsgemäßen Überwachungsverfahrens und
- Figur 3: ein Ablaufdiagramm zu einer zweiten Ausführungsform eines erfindungsgemäßen Überwachungsverfahrens.

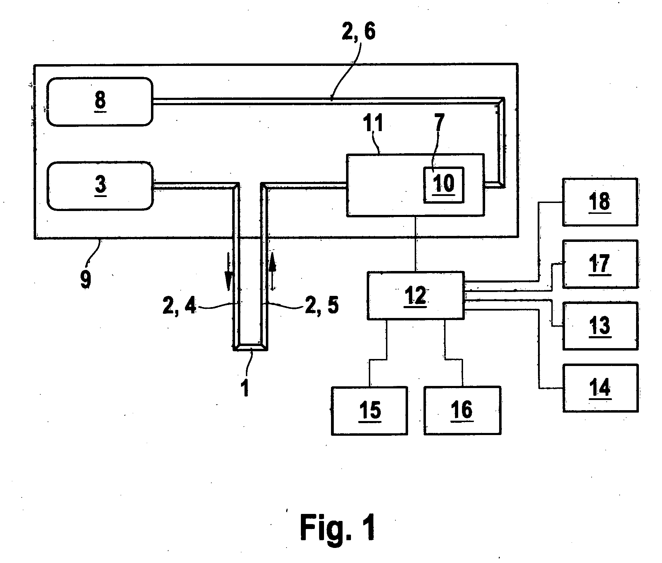

Figur 1 zeigt schematisch die Komponenten einer erfindungsgemäßen Anordnung zur Bestimmung einer Konzentration eines Analyten in einer Körperflüssigkeit. Die Anordnung umfasst eine Schnittstelle 1, zum Beispiel eine Mikrodialysesonde, über die ein Analyt (zum Beispiel Glucose) aus einer Körperflüssigkeit (zum Beispiel interstitieller Flüssigkeit) in eine Flüssigkeitsströmung 2 in der Anordnung gelangen kann (Probengewinnung). Die Perfusionsflüssigkeit wird aus einem Reservoir 3 durch eine erste Flüssigkeitsleitung 4 zu der Schnittstelle 1 hin und von dort aus durch eine zweite Flüssigkeitsleitung 5, eine Durchflussmesskammer 10 und eine dritte Flüssigkeitsleitung 6 in einen Auffangbehälter 8 gepumpt. Zum Pumpen der Flüssigkeit ist eine Transportvorrichtung 9 vorgesehen. In der Durchflussmesskammer 10 befindet sich eine Detektoranordnung 7 (zum Beispiel ein 3-Elektrodensystem enthaltend eine Referenzelektrode, eine Arbeitselektrode und eine Gegenelektrode mit einem Potentiostat), die zur Durchführung von Messungen zur Bestimmung der Konzentration des Analyten dient. Im Bereich der Durchflussmesskammer 10 ist ein Sensorsystem 11 vorgesehen, das zum Messen von Werten mindestens zweier miteinander korrelierter Systemparameter dient. Das Sensorsystem 11 und die Detektoranordnung 7 sind mit einer Signalverarbeitungseinrichtung 12 verbunden. Aus den Ergebnissen der Messungen durch die Detektoranordnung 7 berechnet die Signalverarbeitungseinrichtung 12 (ggf. unter Berücksichtigung eines Kalibrationswertes) die Konzentration des Analyten. Die Zusammenhänge zwischen dem Messsignal, der Konzentration des Analyten in der Flüssigkeitsströmung 2, der Konzentration des Analyten in der Körperflüssigkeit, mit der die Schnittstelle 1 in Kontakt ist (zum Beispiel interstitieller Flüssigkeit), und gegebenenfalls der Konzentration des Analyten in einer weiteren Körperflüssigkeit (zum Beispiel Blut) sind im Stand der Technik bekannt und sind in der Signalverarbeitungseinrichtung 12 vorzugsweise in Form von Berechnungsvorschriften hinterlegt.

Die Signalverarbeitungseinrichtung 12 ist mit einem Speicher 13 verbunden, in dem zum Beispiel gemessene oder berechnete Werte abgelegt werden können. Ferner kann eine weitere Speichereinheit 14 vorgesehen werden, die Referenzwerte zum Vergleichen mit den Messwerten des Sensorsystems 11 enthält. Als Referenzwerte können in der weiteren Speichereinheit 14 Grenzwerte für die mindestens zwei ermittelten Systemparameter hinterlegt werden, wobei hierfür auch der Speicher 13 genutzt werden kann. Die Signalverarbeitungseinrichtung 12 dient als Mittel zum Vergleichen der aktuellen Werte der mindestens zwei miteinander korrelierten Systemparameter mit diesen in der Speichereinheit 14 hinterlegten Grenzwerten. Daraus ergibt sich jeweils eine aktuelle Kombination von mindestens zwei Vergleichsergebnissen. Bei definierten Kombinationen von Vergleichsergebnissen löst die Signalverarbeitungseinrichtung 12 eine Reaktion der Anordnung aus. Auf einer Anzeigeeinrichtung 15 wird beispielsweise als Reaktion eine Warnung (zum Beispiel vor einem erhöhten Messfehler der Detektoranordnung 7), eine Problemmeldung, eine Rekalibrationsaufforderung oder eine Aufforderung, eine Komponente der Anordnung auszutauschen, angezeigt. Über ein Interface 16 (zum Beispiel eine Tastatur) kann ein Benutzer beispielsweise Einfluss auf Einstellungen oder Funktionen der Anordnung nehmen oder einen Rekalibrationswert eingeben. Weitere mögliche Reaktionen auf eine aktuelle Kombination von mindestens zwei Vergleichsergebnissen kann das Ansteuern eines Alarmtongebers 17 sein, der ein akustisches Alarmsignal abgibt. Ferner kann eine Regelung 18 zum Beispiel zur Regelung eines Systemparameters wie der Zellspannung oder der Durchflussrate aktiviert werden. Eine solche Regelung hat den Vorteil, dass ein Betrieb der Anordnung ohne Intervention des Benutzers über einen längeren Zeitraum ermöglicht wird.

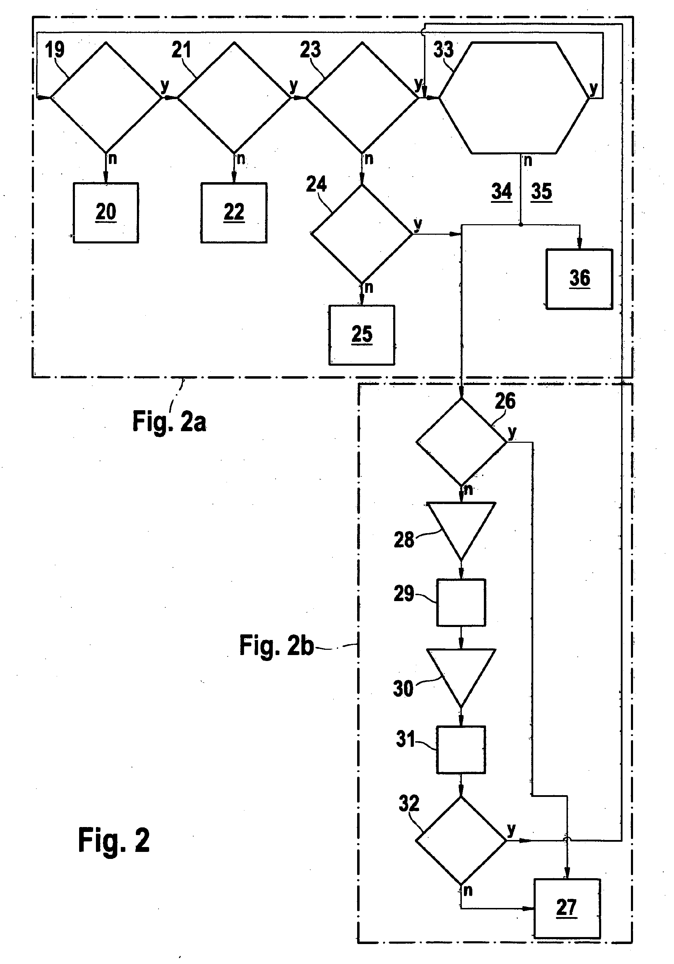

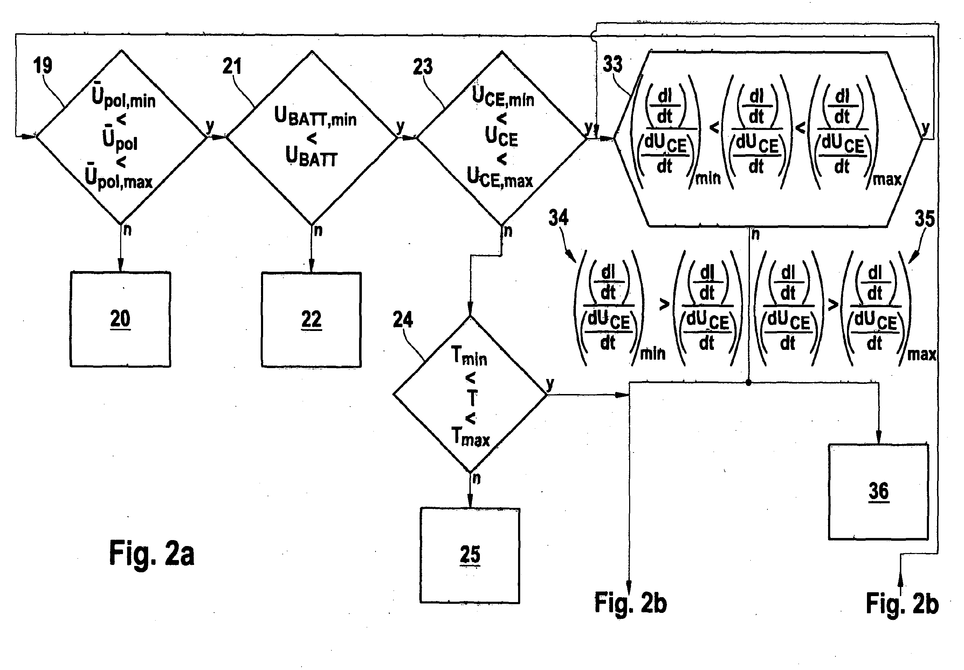

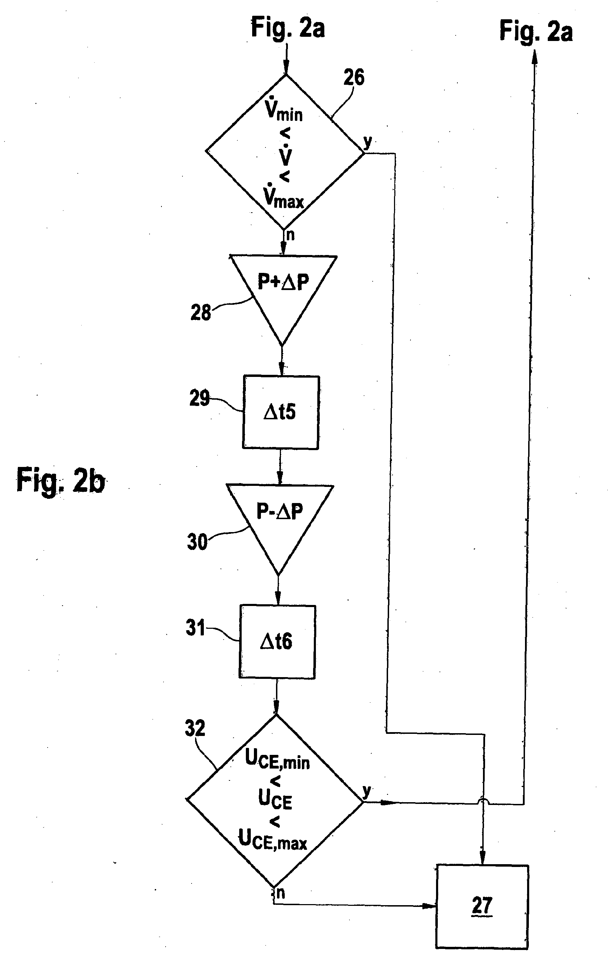

Figur 2 zeigt ein Ablaufdiagramm zu einer ersten Ausführungsform eines erfindungsgemäßen Überwachungsverfahrens.

Dieses Überwachungsverfahren dient zur Überwachung der Sensorspannungen und -ströme einer amperometrischen Detektoranordnung. Dabei wird die Zellspannung (Polarisationsspannung) zwischen der Arbeits- und der Referenzelektrode überwacht durch einen Vergleich 19 von aus Messwerten eines Spannungssensors des Sensorsystems ermittelten mittleren Zellspannungswerten U̅ₚₒₗ mit Grenzwerten U̅_{pol,min} und U̅_{pol,max}. Liegt die Zellspannung außerhalb des durch die Grenzwerte festgelegten Bereichs (Vergleichsergebnis n), so ist davon auszugehen, dass ein Problem seitens der Anordnung oder der Kopplung zwischen Anordnung und Spannungssensor vorliegt. Daher wird ein Alarm ausgelöst/eine Warnmeldung angezeigt 20, um die Beseitigung des Problems durch einen qualifizierten Benutzer zu veranlassen.

Falls der Vergleich 19 ergibt, dass die Zellspannungswerte innerhalb des durch die Grenzwerte festgelegten Bereichs liegen (Vergleichsergebnis y), so wird die Batteriespannung U_{BATT} überprüft. Dabei wird die mit einem Spannungssensor des Sensorsystems gemessene Batteriespannung mit einem unteren Grenzwert U_{BATT,min} verglichen (Bezugszeichen 21). Liegt die Batteriespannung unter dem Grenzwert (Vergleichsergebnis n), so wird ein Alarm ausgelöst/eine Meldung angezeigt 22, um den Benutzer zu einem Batteriewechsel aufzufordern.

Falls der Vergleich 21 ergibt, dass die Batteriespannungswerte über dem unteren Grenzwert liegen (Vergleichsergebnis y), so wird die Gegenspannung U_{CE} zwischen Gegen- und Arbeitselektrode der Detektoranordnung überprüft. Dazu wird die mit einem Spannungssensor des Sensorsystems gemessene Gegenspannung mit unteren und oberen Grenzwerten U_{CE,min} bzw. U_{CE,max} verglichen (Bezugszeichen 23). Liegt die Gegenspannung außerhalb des durch die Grenzwerte definierten Bereichs (Vergleichsergebnis n), so wird erfindungsgemäß die mit der Gegenspannung korrelierte Temperatur T überprüft durch einen Vergleich 24 mit unteren und oberen Grenzwerten Tₘᵢₙ und Tₘₐₓ. Falls die Temperatur, die mit einem Temperatursensor des Sensorsystems gemessen wird, außerhalb des durch die Grenzwerte festgelegten Bereichs liegt (Vergleichsergebnis n), so ergibt sich aus der Kombination der Vergleichsergebnisse zu Gegenspannung und Temperatur (n, n), dass ein Systemfehler vorliegt und ein Alarm 25 auszulösen ist.

Falls die Temperatur innerhalb des durch die Grenzwerte definierten Bereichs liegt (Vergleichsergebnis y), so wird erfindungsgemäß die mit der Gegenspannung korrelierte mittlere Durchflussrate *V̇* durch Vergleich 26 mit Grenzwerten *V̇*ₘᵢₙ und *V̇*ₘₐₓ überprüft. Falls die mittlere Durchflussrate *V̇*, die aus Messwerten eines Durchflussratensensors des Sensorsystems ermittelt wird, innerhalb des durch die Grenzwerte festgelegten Bereichs liegt (Vergleichsergebnis y), so ergibt sich aus der Kombination der Vergleichsergebnisse zur Gegenspannung, Temperatur und mittleren Durchflussrate (n, y, y), dass ein Systemfehler vorliegt und ein Alarm 27 auszulösen ist.

Falls die mittlere Durchflussrate *V̇* außerhalb des durch die Grenzwerte festgelegten Bereichs liegt (Vergleichsergebnis n), so ergibt sich aus der Kombination der Vergleichsergebnisse zur Gegenspannung, Temperatur und mittleren Durchflussrate (n, y, n), dass gegebenenfalls ein Flusshindernis zu einer zumindest teilweisen Belegung der Gegenelektrode führt. Zwecks Beseitigung wird daher als Reaktion für eine Zeitspanne Δt5 eine Pumpvariable P (zum Beispiel der elektrische Pumpenstrom) um ΔP erhöht (Bezugszeichen 28), um die Durchflussrate zu erhöhen. Nach Verstreichen von Δt5 (Bezugszeichen 29) wird die Pumpenvariable wieder um ΔP gesenkt (Bezugszeichen 30). Anschließend wird nach Verstreichen einer Zeitspanne Δt6 (Bezugszeichen 31) erneut ein Vergleich 32 der Gegenspannung U_{CE} mit den Grenzwerten U_{CE,min} und U_{CE,max} vorgenommen.

Falls der Vergleich 32 ergibt, dass die Gegenspannung weiterhin außerhalb des durch die Grenzwerte definierten Bereichs liegt (Vergleichsergebnis n), so wird als Reaktion ein Alarm 27 ausgelöst. Falls sie nun innerhalb des Bereichs liegt (Vergleichsergebnis y), so wird als nächstes das Verhältnis der zeitlichen Änderung des Messstroms dI/dt zu der zeitlichen Änderung der Gegenspannung dU_{CE}/dt überprüft durch Vergleich 33 mit unteren und oberen Grenzwerten. Dieser Vergleich wird auch dann durchgeführt, wenn der ursprüngliche Vergleich 23 der Gegenspannung das Vergleichsergebnis y geliefert hat (Gegenspannung liegt zwischen den Grenzwerten). Wenn das Verhältnis der zeitlichen Änderung des Messstroms zur zeitlichen Änderung der Gegenspannung zwischen den Grenzwerten liegt (Vergleichsergebnis y), so wird das Verfahren erneut beginnend mit dem Vergleich 19 der Zellspannung durchgeführt. Falls das Verhältnis außerhalb des durch die Grenzwerte festgelegten Bereichs liegt, wird zwischen zwei Fällen 34, 35 unterschieden. Im ersten Fall 34 wird der untere Grenzwert unterschritten. Als Reaktion wird das Verfahren - wie zuvor beschrieben - mit dem Überprüfen der Durchflussrate (Vergleich 26) fortgesetzt. Im zweiten Fall 35 wird der obere Grenzwert überschritten und als Reaktion wird die Anordnung abgeschaltet 36, da der Verdacht eines Kurzschlusses besteht.

Die Figur 2 mit weiteren Beschriftungen ist vergrößert in den beiden Teilfiguren 2a und 2b dargestellt.

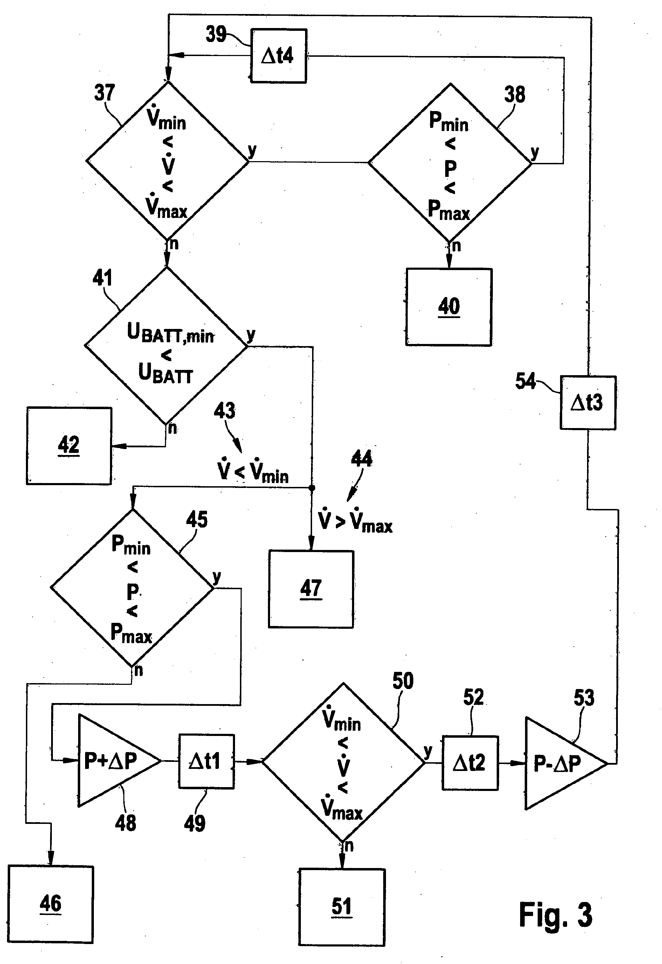

Figur 3 zeigt ein Ablaufdiagramm zu einer zweiten Ausführungsform eines erfindungsgemäßen Überwachungsverfahrens.

Die Ausführungsformen gemäß Figuren 2 und 3 sind in einer erfindungsgemäßen Anordnung (zum Beispiel gemäß Figur 1) einzeln oder in Kombination ausführbar. Das Überwachungsverfahren gemäß Figur 3 dient der Überwachung der Durchflussrate in einer Anordnung zur Bestimmung der Konzentration eines Analyten.

Die Konstanz der Durchflussrate einer Flüssigkeitsströmung ist eine wichtige Voraussetzung für eine konstante Totzeit, einen konstanten Stoffaustausch über die Schnittstelle (zum Beispiel Membranwandung eines Katheters) und die Konzentrationsmessung in der Durchflussmesskammer (zum Beispiel elektrochemische Messung). Die mittlere Durchflussrate wird daher vorzugsweise durch einen Vergleich 37 einer aus Messwerten eines Durchflussratensensors ermittelten Durchflussrate *V̇* mit unteren und oberen Grenzwerten *V̇* ₘᵢₙ bzw. *V̇* ₘₐₓ überwacht. Liegt die Durchflussrate in dem durch die Grenzwerte definierten Bereich (Vergleichsergebnis y), so wird die mit der Durchflussrate korrelierte Pumpvariable P zur Überprüfung der Transportvorrichtung (Pumpe) durch einen Vergleich 38 mit unteren und oberen Grenzwerten Pₘᵢₙ bzw. Pₘₐₓ überprüft. Im Falle einer zwischen den Grenzwerten liegenden Pumpvariable (Vergleichsergebnis y) ergibt sich aus der Kombination der Vergleichsergebnisse von Durchflussrate und Pumpenvariablen (y, y), dass die Transportvorrichtung (Pumpe) korrekt funktioniert. Dann wird der Vergleich 37 der aktuellen Durchflussrate mit den Grenzwerten nach einer Zeitspanne Δt4 (Bezugszeichen 39) wiederholt.

Im Falle einer in dem Vergleich 38 außerhalb des durch die Grenzwerte definierten Bereichs liegenden Pumpenvariable (Vergleichsergebnis n) (zum Beispiel bei zu hoher Stromaufnahme der Pumpe) wird ein Alarm ausgelöst/eine Meldung angezeigt 40, um einen Benutzer zum Überprüfen der Pumpe aufzufordern.

Ergibt der Vergleich 37 der mittleren Durchflussrate, dass diese außerhalb des durch die Grenzwerte festgelegten Bereichs liegt (Vergleichsergebnis n), so wird die mit der mittleren Durchflussrate korrelierte Batteriespannung U_{BATT} überprüft. Sie wird dazu mit einem unteren Grenzwert U_{BATT,min} verglichen (Bezugszeichen 41). Unterschreitet die Batteriespannung den Grenzwert (Vergleichsergebnis n), so wird der Benutzer durch einen Alarm oder eine Meldung 42 zum Austausch der Batterie/des Akkus aufgefordert. Falls die Batteriespannung über dem Grenzwert liegt (Vergleichsergebnis y), so werden zwei Fälle 43, 44 für diese Kombination von Vergleichsergebnissen der Flussrate und der Batteriespannung (n, y) unterschieden.

Im ersten Fall 43 liegt die mittlere Durchflussrate unter dem unteren Grenzwert *V̇* ₘᵢₙ. In diesem Fall wird die mit der Durchflussrate korrelierte Pumpenvariable P (zum Beispiel Pumpenstrom oder Drehzahl) überprüft durch Vergleich 45 mit Grenzwerten Pₘᵢₙ und Pₘₐₓ. Falls die Pumpenvariable außerhalb des durch die Grenzwerte festgelegten Bereichs liegt (Vergleichsergebnis n), so ergibt sich aus der Kombination der Vergleichsergebnisse zur mittleren Durchflussrate, Batteriespannung und Pumpenvariable (n, y, n), dass ein Defekt der Transportvorrichtung (Pumpe) vorliegt, so dass ein Alarm ausgelöst/eine Meldung angezeigt wird 46, um ein Überprüfen der Pumpe zu veranlassen.

Falls die Pumpenvariable in dem durch die Grenzwerte definierten Bereich liegt (Vergleichsergebnis y im Vergleich 45), so ergibt sich aus der Kombination der Vergleichsergebnisse zur mittleren Durchflussrate, Batteriespannung und Pumpenvariablen (n, y, y), dass gegebenenfalls eine Blase oder ein Partikel ein temporäres Flusshindernis bildet. Zwecks Beseitigung wird daher als Reaktion für eine Zeitspanne Δt1 die Pumpenvariable P (zum Beispiel der Pumpenstrom) um ΔP erhöht (Bezugszeichen 48), um die Durchflussrate zu erhöhen. Nach Verstreichen von Δt1 (Bezugszeichen 49) wird erneut ein Vergleich 50 der mittleren Durchflussrate mit den Grenzwerten *V̇*ₘᵢₙ und *V̇*ₘₐₓ vorgenommen.

Falls der Vergleich 50 ergibt, dass die mittlere Durchflussrate weiterhin außerhalb des durch die Grenzwerte definierten Bereichs liegt (Vergleichsergebnis n), so wird als Reaktion ein Alarm ausgelöst oder eine Warnmeldung angezeigt (Bezugszeichen 51). Falls sie innerhalb des Bereichs liegt (Vergleichsergebnis y), so wird nach Verstreichen einer Zeitspanne Δt2 (Bezugszeichen 52) die Pumpenvariable wieder um ΔP gesenkt (Bezugszeichen 53). Danach wird die mittlere Durchflussrate nach Ablauf einer definierten Zeitspanne Δt3 (Bezugszeichen 54) erneut überprüft (Vergleich 37).

Im zweiten Fall 44 liegt die mittlere Durchflussrate über dem oberen Grenzwert *V̇*ₘₐₓ. In diesem Fall wird direkt ein Alarm 47 ausgelöst, um vor einem Systemfehler zu warnen.

### Bezugszeichenliste

- 1: Schnittstelle
- 2: Flüssigkeitsströmung
- 3: Reservoir
- 4: Erste Flüssigkeitsleitung
- 5: Zweite Flüssigkeitsleitung
- 6: Dritte Flüssigkeitsleitung
- 7: Detektoranordnung
- 8: Auffangbehälter
- 9: Transportvorrichtung
- 10: Durchflussmesskammer
- 11: Sensorsystem
- 12: Signalverarbeitungseinrichtung
- 13: Speicher
- 14: Speichereinheit
- 15: Anzeigeeinrichtung
- 16: Interface
- 17: Alarmtongeber
- 18: Regelung
- 19: Vergleich Zellspannung
- 20: Alarm/Warnmeldung
- 21: Vergleich Batteriespannung
- 22: Alarm/Warnmeldung
- 23: Vergleich Gegenspannung
- 24: Vergleich Temperatur
- 25: Alarm
- 26: Vergleich mittlere Durchflussrate
- 27: Alarm
- 28: Erhöhung Pumpvariable
- 29: Zeitspanne
- 30: Absenken Pumpvariable
- 31: Zeitspanne
- 32: Vergleich Gegenspannung
- 33: Vergleich Verhältnis
- 34: Erster Fall
- 35: Zweiter Fall
- 36: Abschalten der Anordnung
- 37: Vergleich mittlere Durchflussrate
- 38: Vergleich Pumpvariable
- 39: Zeitspanne
- 40: Alarm/Meldung
- 41: Vergleich Batteriespannung
- 42: Alarm/Meldung
- 43: Erster Fall
- 44: Zweiter Fall
- 45: Vergleich Pumpvariable
- 46: Alarm/Meldung
- 47: Alarm
- 48: Erhöhen der Pumpvariable
- 49: Erste Zeitspanne
- 50: Vergleich mittlere Durchflussrate
- 51: Alarm/Warnmeldung
- 52: Zweite Zeitspanne
- 53: Absenken der Pumpvariable
- 54: Dritte Zeitspanne

## Patentansprüche

1. Verfahren zur Überwachung einer Anordnung zum Bestimmen einer Konzentration eines Analyten einer Körperflüssigkeit, wobei das Bestimmen der Konzentration des Analyten mittels der Anordnung darauf beruht, dass der Analyt aus der Körperflüssigkeit durch eine Schnittstelle hindurchtritt und in einer Flüssigkeitsströmung in eine Durchflussmesskammer transportiert wird, in der eine Messung zur Bestimmung der Konzentration des Analyten durchgeführt wird, und eine Auswertung der Messung in einer Signalverarbeitungseinrichtung erfolgt, **dadurch gekennzeichnet, dass** die Überwachung der Anordnung die Schritte umfasst
• Messen von Messwerten mindestens zweier miteinander korrelierter Systemparameter der Anordnung mittels eines Sensorsystems und
• Vergleichen der Messwerte mit jeweils für die Systemparameter in einer Speichereinheit hinteren Grenzwerten zum Erhalt einer Kombination von mindestens zwei Vergleichsergebnissen.

2. Verfahren gemäß Anspruch 1, **dadurch gekennzeichnet, dass** die Signalverarbeitungseinrichtung bei definierten Kombinationen von Vergleichsergebnissen mindestens eine Reaktion der Anordnung auslöst, wobei die mindestens eine Reaktion ausgewählt ist aus der Gruppe
• Anzeigen einer Warnung oder Problemmeldung durch eine Anzeigeeinrichtung der Anordnung,
• Anzeigen einer Rekalibrationsaufforderung durch die Anzeigeeinrichtung,
• Anzeigen einer Aufforderung, eine Komponente der Anordnung auszutauschen, durch die Anzeigeeinrichtung,
• Abschalten der Anordnung,
• Abgabe eines optischen, akustischen oder haptischen Alarmsignals,
• Regeln mindestens eines Systemparameters,
• Beeinflussen von Werten eines ersten Systemparameters durch Regeln von Werten eines zweiten, mit dem ersten Systemparameter korrelierten Systemparameters,
• kurzzeitiges Ändern eines Systemparameters zur Behebung eines aufgrund der Kombination der Vergleichsergebnisse möglichen Systemfehlers.

3. Verfahren gemäß einem der Ansprüche 1 oder 2, **dadurch gekennzeichnet, dass** das Sensorsystem Messungen zur Bestimmung mindestens zweier Systemparameter, ausgewählt aus der Gruppe
• Temperatur der Flüssigkeitsströmung,
• elektrische Spannung zwischen mindestens zwei Elektroden, an denen die Flüssigkeitströmung in der Durchflussmesskammer entlangströmt,
• zeitliche Änderung eines elektrischen Stroms im Verhältnis zu einer zeitliche Änderung einer elektrischen Spannung in Bezug auf mindestens zwei in der Durchflussmesskammer angeordnete Elektroden,
• Systemvariable einer in der Anordnung enthaltenen Transportvorrichtung, die zum Transport der Flüssigkeitsströmung dient,
• Batteriespannung mindestens einer in der Anordnung zur Energieversorgung vorgesehenen Batterie,
• Durchflussrate der Flüssigkeitsströmung durch die Purchflussmess-kammer und
• Konzentration eines Markers,
durchführt.

4. Verfahren gemäß einem der Ansprüche 1 bis 3, **dadurch gekennzeichnet, dass** eine Gegenspannung zwischen einer in der Durchflussmesskammer angeordneten Gegen- und einer Arbeitselektrode, eine Temperatur der Flüssigkeitsströmung und eine Durchflussrate der Flüssigkeitsströmung durch die Durchflussmesskammer jeweils durch die Signalverarbeitungseinrichtung mit in der Speichereinheit hinterlegten Grenzwerten verglichen werden.

5. Verfahren gemäß einem der Ansprüche 1 bis 4, **dadurch gekennzeichnet, dass** eine Durchflussrate der Flüssigkeitsströmung durch die Durchflussmesskammer, eine Batteriespannung einer in der Anordnung enthaltenen Batterie und eine Systemvariable einer in der Anordnung enthaltenen Transportvorrichtung, die zum Transport der Flüssigkeitsströmung dient, jeweils durch die Signalverarbeitungseinrichtung mit in der Speichereinheit hinterlegten Grenzwerten verglichen werden.

6. Verfahren gemäß einem der Ansprüche 1 bis 5, **dadurch gekennzeichnet, dass** Werte mindestens einer durch das Sensorsystem gemessenen Systemvariablen bei der Bestimmung der Konzentration des Analyten berücksichtigt werden.

7. Verfahren gemäß einem der Ansprüche 1 bis 6, **dadurch gekennzeichnet, dass** die Messung zur Bestimmung der Konzentration des Analyten mit einer definierten Messfrequenz erfolgt, und dass das Messen der Messwerte der mindestens zwei, miteinander korrelierten Systemparameter ebenfalls mit der definierten Messfrequenz erfolgt.

8. Anordnung zum Bestimmen einer Konzentration eines Analyten in einer Körperflüssigkeit, umfassend eine Schnittstelle (1) zum Aufnehmen des Analyten aus einer Körperflüssigkeit in eine Flüssigkeitsströmung in der Anordnung, eine Flüssigkeitsleitung (5) zum Leiten der Perfusionsflüssigkeitsströmung (2) von der Schnittstelle (1) zu einer Durchflussmesskammer (10), eine Transportvorrichtung (9) zum Transport der Flüssigkeitsströmung (2) von der Schnittstelle (1 ) zu der Durchflussmesskammer (10), eine Detektoranordnung (7) in der Durchflussmesskammer (10) zur Durchführung von Messungen zum Bestimmen der Konzentration des Analyten und eine Signalverarbeitungseinrichtung (12) zur Berechnung der Konzentration des Analyten aus Ergebnissen der Messungen der Detektoranordnung (7), **gekennzeichnet durch** ein sensorsystem (11) zum Messen von Messwerten mindestens zweier miteinander korrelierter Systemparameter, eine Speichereinheit (14) und Mittel zum Vergleichen der Messwerte mit jeweils für die Systemparameter in der Speichereinheit (14) hinterlegten Grenzwerten zum Erhalt einer Kombination von mindestens zwei Vergleichsergebnissen.

9. Anordnung gemäß Anspruch 8, **dadurch gekennzeichnet, dass** das Sensorsystem (11) mindestens zwei Sensoren ausgewählt aus der Gruppe elektrischer Spannungssensor, elektrischer Stromsensor, Temperatursensor, Durchflussratensensor, Drehzahlmesser und Konzentrationssensor umfasst.

10. Verwendung eines Sensorsystems zum Messen von Messwerten mindestens zweier miteinander korrelierten Systemparameter zur Überwachung einer Anordnung gemäß einem der Ansprüche 8 oder 9, wobei die Anordnung zum Bestimmen der Konzentration von Glucose in einer Körperflüssigkeit dient.

## Claims

1. Process for monitoring an arrangement for determining a concentration of an analyte of a body fluid, wherein the determination of the concentration of the analyte by means of the arrangement is based on the fact that the analyte from the body fluid passes through an interface and is transported in a liquid stream into a flow measurement chamber in which a measurement is carried out in order to determine the concentration of the analyte, and an evaluation of the measurement takes place in a signal processing device, **characterised in that** the monitoring of the arrangement comprises the following steps:
• determination of measurement values of at least two mutually correlated system parameters of the arrangement by means of a sensor system, and
• comparison of the measurement values with in each case limiting values for the system parameters stored in a memory unit so as to obtain a combination of at least two comparison results.

2. Process according to claim 1, **characterised in that** the signal processing device triggers at defined combinations of comparison results at least one response of the arrangement, wherein the at least one response is selected from the group comprising
• display of a warning or problem notification by a display device of the arrangement,
• display of a recalibration request by the display device,
• display of a request to interchange a component of the arrangement, by the display device,
• switching off of the arrangement,
• emission of an optical, acoustic or haptic alarm signal,
• regulation of at least one system parameter,
• influencing of values of a first system parameter by regulating values of a second system parameter correlated with the first system parameter,
• brief alteration of a system parameter in order to rectify a system error possibly arising on account of the combination of the comparison results.

3. Process according to one of claims 1 and 2, **characterised in that** the sensor system carries out measurements to determine at least two system parameters selected from the following group:
• temperature of the liquid stream,
• electrical voltage between at least two electrodes over which the liquid stream flows in the flow measurement chamber;
• time change of an electrical current in comparison to a time change of an electrical voltage with respect to at least two electrodes arranged in the flow measurement chamber,
• system variables of a transporting device contained in the arrangement, which serves to transport the liquid stream,
• battery voltage of at least one battery provided in the arrangement for the energy supply,
• flow rate of the liquid stream through the flow measurement chamber, and
• concentration of a marker.

4. Process according to one of claims 1 to 3, **characterised in that** a countervoltage between a counterelectrode and an operating electrode arranged in the flow measurement chamber, a temperature of the liquid stream, and a flow rate of the liquid stream through the flow measurement chamber are in each case compared by the signal processing device with limiting values stored in the memory unit.

5. Process according to one of claims 1 to 4, **characterised in that** a flow rate of the liquid stream through the flow measurement chamber, a battery voltage of a battery contained in the arrangement, and a system variable of a transporting device contained in the arrangement and serving to transport the liquid stream, are in each case compared by the signal processing device with limiting values stored in the memory unit.

6. Process according to one of claims 1 to 5, **characterised in that** values of at least one system variable measured by the sensor system are taken into account in the determination of the concentration of the analyte.

7. Process according to one of claims 1 to 6, **characterised in that** the measurement for determining the concentration of the analyte takes place at a defined measurement frequency, and that the measuring of the measurement values of the at least two mutually correlated system parameters likewise takes place at the defined measurement frequency.

8. Arrangement for determining a concentration of an analyte in a body fluid, comprising an interface (1) for receiving the analyte from a body fluid in a liquid stream in the arrangement, a liquid line (5) for conveying the perfusion liquid stream (2) from the interface (1) to a flow measurement chamber (10), a transporting device (9) for transporting the liquid stream (2) from the interface (1) to the flow measurement chamber, a detector arrangement (7) in the flow measurement chamber (10) for carrying out measurements in order to determine the concentration of the analyte, and a signal processing device (12) for calculating the concentration of the analyte from results of the measurements performed by the detector arrangement, **characterised by** a sensor system (11) for measuring measurement values of at least two mutually correlated system parameters, a memory unit (14), and means for comparing the measurement values with in each case limiting values for the system parameters stored in the memory unit (14), in order to obtain a combination of at least two comparison results.

9. Arrangement according to claim 8, **characterised in that** the sensor system (11) includes at least two sensors selected from the group comprising electrical voltage sensor, electrical current sensor, temperature sensor, flow rate sensor, rotational speed measurement device and concentration sensor.

10. Use of a sensor system for measuring measurement values of at least two mutually correlated system parameters for monitoring an arrangement according to one of claims 8 and 9, wherein the arrangement serves to determine the concentration of glucose in a body fluid.

## Revendications

1. Procédé pour surveiller une configuration destinée à déterminer une concentration d'un analyte d'un liquide corporel, la détermination de la concentration de l'analyte au moyen de la configuration se basant sur le fait que l'analyte issu du liquide corporel traverse une interface et est transporté dans un courant de liquide dans une chambre de mesure de débit, dans laquelle une mesure est effectuée pour déterminer la concentration de l'analyte, et une évaluation de la mesure se réalise dans un dispositif de traitement de signaux, **caractérisé en ce que** la surveillance de la configuration comprend les étapes consistant à :
• la mesure des valeurs expérimentales d'au moins deux paramètres de système corrélés entre eux de la configuration au moyen d'un système de capteurs, et
• la comparaison des valeurs expérimentales à des valeurs limites pour les paramètres de système, mémorisés à chaque fois dans une unité de mémoire pour chaque paramètre de système afin d'obtenir une combinaison d'au moins deux résultats de comparaison.

2. Procédé selon la revendication 1, **caractérisé en ce que** le dispositif de traitement de signaux pour des combinaisons définies de résultats de comparaison, déclenche au moins une réaction de la configuration, ladite au moins une réaction étant choisie dans le groupe formé par :
• l'affichage d'un avertissement ou d'une annonce de problème à l'aide d'un dispositif d'affichage de la configuration,
• l'affichage d'une demande de réétalonnage à l'aide du dispositif d'affichage,
• l'affichage d'une demande de remplacement d'un composant de la configuration, à l'aide du dispositif d'affichage,
• la mise hors service de la configuration,
• l'émission d'un signal d'alarme optique, acoustique, ou haptique,
• l'ajustement d'au moins un paramètre de système,
• le fait d'influencer des valeurs d'un premier paramètre de système à l'aide d'un réglage de valeurs d'un deuxième paramètre système corrélé au premier paramètre de système,
• la modification momentanée d'un paramètre de système pour éliminer une erreur de système possible en raison de la combinaison des résultats de comparaison.

3. Procédé selon l'une quelconque des revendications 1 ou 2, **caractérisé en ce que** le système de capteurs réalise des mesures pour déterminer au moins deux paramètres de système, choisis dans le groupe formé par
• une température du courant de liquide,
• une tension électrique entre au moins deux électrodes sur lesquelles et le long desquelles coule le courant de liquide dans la chambre de mesure de débit,
• une modification temporaire d'un courant électrique en proportion à une modification temporaire d'une tension électrique par rapport à au moins deux électrodes disposées dans la chambre de mesure de débit,
• une variable de système d'un dispositif de transport contenu dans la configuration, qui sert au transport du courant de liquide,
• une tension de batterie d'au moins une batterie prévue dans la configuration pour l'alimentation en énergie,
• un débit du courant liquide à travers la chambre de mesure de débits, et
• une concentration d'un marqueur.

4. Procédé selon l'une quelconque des revendications 1 à 3, **caractérisé en ce qu'**une contre-tension entre une contre-électrode disposée dans la chambre de mesure de débit et une électrode de travail, une température du courant de liquide et un débit du courant de liquide à travers la chambre de mesure de débit sont comparées à chaque fois par le dispositif de traitement de signaux aux valeurs limites mémorisées dans l'unité mémoire.

5. Procédé selon l'une quelconque des revendications 1 à 4, **caractérisé en ce qu'**un débit du courant de liquide à travers la chambre de mesure de débit, une tension de batterie d'une batterie contenue dans la configuration et une variable de système d'un dispositif de transport contenu dans la configuration, qui sert au transport du courant de liquide, sont comparés à chaque fois par le dispositif de traitement de signaux aux valeurs limite mémorisées dans l'unité de mémoire.

6. Procédé selon l'une quelconque des revendications 1 à 5, **caractérisé en ce que** des valeurs d'au moins une variable de système mesurée par le système de capteurs sont prises en compte pour la détermination de la concentration de l'analyte.

7. Procédé selon l'une quelconque des revendications 1 à 6, **caractérisé en ce que** la mesure pour déterminer la concentration de l'analyte se réalise avec une fréquence de mesure définie, et que la mesure des valeurs expérimentales desdits au moins deux paramètres de système corrélés entre eux, se réalise également avec la fréquence de mesure définie.

8. Configuration pour déterminer une concentration d'un analyte dans un liquide corporel, comprenant une interface (1) pour l'admission de l'analyte issu d'un liquide corporel, dans un courant de liquide dans la configuration, une conduite de liquide (5) pour conduire le courant de liquide de perfusion (2) à partir de l'interface (1) vers une chambre de mesure de débit (10), un dispositif de transport (9) pour transporter le courant de liquide (2) à partir de l'interface (1) vers la chambre de mesure de débit (10), une configuration de détecteurs (7) dans la chambre de mesure de débit (10) pour réaliser des mesures pour déterminer la concentration de l'analyte et un dispositif de traitement de signaux (12) pour calculer la concentration de l'analyte d'après les résultats des mesures de la configuration de détecteurs (7), **caractérisée par** un système de capteurs (11) pour mesurer des valeurs de mesure d'au moins deux paramètres système corrélés entre eux, par une unité mémoire (14) et des moyens pour comparer les valeurs expérimentales aux valeurs limites pour les paramètres de système mémorisés dans l'unité de mémoire (14) pour chaque paramètre de système pour obtenir une combinaison d'au moins deux résultats de comparaison.

9. Configuration selon la revendication 8, **caractérisée en ce que** le système de capteurs (11) comprend au moins deux capteurs choisis dans le groupe formé par les capteurs de tension électrique, les capteurs de courant électrique, les capteurs de température, les capteurs de débit, les tachymètres et les capteurs de concentration.

10. Utilisation d'un système de capteurs pour mesurer des valeurs expérimentales d'au moins deux paramètres de système corrélés entre eux pour surveiller une configuration selon l'une quelconque des revendications 8 ou 9, la configuration servant à déterminer la concentration de glucose dans un liquide corporel.
